# EUROPEAN PATENT APPLICATION

(11) **EP 1 464 304 A2**
(43) Date of publication of application: **06.10.2004**
(21) Application number: 04007826.3
(22) Date of filing: 31.03.2004
(51) Int. Cl.: A61F 2/36

(54) **Prosthetic device**

(30) Priority: 01.04.2003 ES 200300764
(71) Applicant: IQL Industria Quirurgicas de Levante S.L., Paterna (Valencia) (ES)
(72) Inventor: Couceiro Follente, José, Dr., 46988 Fuente Del Jarro Valencia (ES); Carreres Sancho, Benjamin, 46988 Fuente Del Jarro Valencia (ES)
(74) Representative: Koepe, Gerd L., Dipl.-Chem.

(57) **Abstract**

The present invention relates to a prosthesis (1) for implantation into a hollow canal of a bone, said prosthesis comprising
- a shaft member (10) having a longitudinal axis (12) and having a distal end (16), a proximal end (14), an anterior side (22), a posterior side (24), a medial side (26) and a lateral side (28), said shaft member (10) widening conically from said distal end (16) to said proximal end (14) and from said medial side (26) to said lateral side (28), and having a proximal locking zone (34) comprising a number of proximal locking surfaces (62A, 62B, 62C, 62D, ....); and
- a neck (18) for receiving a head portion of the prosthesis, said neck (18) extending from said proximal end (14) of said shaft member (10) in an angle (α), relative to said longitudinal axis (12), and having a peg portion (52), a first circumferential fillet (56), a second circumferential fillet (58) and a chamfer (60);
- wherein the proximal taper of the proximal locking surfaces (62B, 62D) is within a range of from 10 to 20 °, relative to the longitudinal axis (12).

## Description

### Field of the Invention

The present invention relates generally to a prosthetic device and relates, in particular, to a prosthetic device of a component of a hip joint prosthesis for implantation into the femur bone so as to replace a natural hip joint and proximal part of the femur bone.

### Background of the Invention

Prosthetic devices are used for replacing load-carrying skeletal members, such as the human hip, which are rendered non-functional or worsened in function due to acute arthritis, fracture, resections for malignancy or malformation. Surgery for replacing hip joints has become more common not only in human beings but also in animals like dogs or cats.

Hip joints are usually repaired by total hip joint replacement with artificial components of a hip joint prosthesis. Such hip prostheses typically include a femur portion or component which is implanted into the femur and an acetabular component which is secured to the pelvis. The femur portion includes a head which articulates in a socket formed in the acetabular component.

Due to the load carried by the hip joint, it is desirable in cases where the natural joint is replaced by a prosthesis that there is provided a rigid fixation of the prosthesis in the femur in order to achieve a long-term stabilization, to minimize bone-implant micromotion and to minimize the occurrence of complications such as pain after surgery or loosening due to bad design conditions and wrong force distribution between implant and bone.

Known hip prosthesis according to the prior art achieve a rigid fixation of the prosthesis through the use of a cement for embedding the prosthesis into the bone structure, particularly into the femur. Numerous disadvantages encountered with prostheses of said type were overcome by the development of implants, particularly hip joint prosthesis implants, which are inserted into femur intramedullary bone canals so as to obtain a cementless press-fit arrangement.

A femur component for a hip joint prosthesis of the type to which the present invention relates is known from the U. S. Patent No. 2,719,522. In said document, there is disclosed a prosthesis device having a shaft in the form of a truncated cone which widens continuously from the distal end to the proximal end. Furthermore, this shaft has an additional conical widening with a circular cross section in the proximal shaft area. A shape of this type is only approximately capable of being adjusted to the proximal narrow space of the femur. However, such a shape does not result in optimum anchoring in the proximal area which anchoring is of particular importance for prosthesis shafts which are to be implanted without the use of a cement.

It has also been found in recent years that hip joint prostheses settle after a period of time, i. e. they tend to sink deeper into the bone. Thus, it is important that, during this sinking, the prosthesis immediately again locks and fastens so that, at the boundary surface of the prosthesis to the bone, no micromovements occur which could lead to bone distruction and removal, as is well known. The prosthesis shaft of the above-referenced U. S. Patent enables relocking only during axial sinking of the prosthesis shaft. However, it has been found that sinking of the prosthesis shaft does not only occur in the direction of the longitudinal axis of the shaft, but that, due to the movement which is exerted by the stress acting on the joint head of the femur component with regard to the longitudinal axis, a medially directed sinking does occur, particularly in the proximal area of the shaft. Furthermore, torsion stability between stem and bone is essential. The implant anchorage needs to distribute rotational forces accordingly to the surrounding tissue.

The U. S. Patent No. 4,728,334 discloses a femur component of a hip joint prosthesis which comprises a straight shaft member defining a longitudinal axis and having a distal end, a proximal end, an anterior side, a posterior side, a medial side and a lateral side, wherein the shaft member is formed, at least over a length dimension which extends near the proximal end of the shaft, with a conical configuration which widens, taken in the direction towards the proximal end of the shaft, and with a configuration which narrows medially, taken from the lateral side to the medial side of the shaft. In a proximal portion, the shaft member has a plurality of distinct blades having recesses therebetween, wherein said blades and recesses are disposed substantially parallel to said longitudinal axis. Each said blade has a broad surface extending a distance perpendicularly to the anterior posterior plane of said shaft. In addition, said distance and depth of each of said blades and recesses increases in a direction from said medial to said lateral side to yield the general conical configuration, and said recesses between said blades have a depth which gradually decreases in a direction from said lateral to said medial side.

The U.S. Patent No. 5,863,295 relates to a hip prosthesis for implantation into the medullary canal of a femur, which prosthesis comprises a stem for implanting into the medullary canal of the femur, the stem having a proximal end and a distal end and also including a proximal locking zone substantially adjacent the proximal end, the proximal locking zone including a proximal locking surface which circumferentially press-fits within the canal of the femur, and a neck extending at an angle from the proximal end of the stem for receiving the femoral head of the prosthesis. The stem further defines a longitudinal axis. The proximal locking surface and the longitudinal axis define a proximal locking angle therebetween. This proximal locking angle on the anterior and posterior portions of the proximal locking surface may range between 5 and 10 degrees, with 7 degrees being preferred, which results in a full wedge angle of 10 to 20 degrees, with 12 to 15 degrees being preferred.

The known hip joint implant prostheses for cement-less press-fit implantation still suffer from a number of disadvantages. Particularly, the tendency to sink deeper into the medullary canal after a certain time of loading during natural movement was undesired due to the uncertain change of position of the stem or shaft within the canal. In addition, the load exerted by the prosthesis head onto the axis of the stem or shaft resulted into torsions and axial instability which deteriorated the seat of the prosthesis stem or shaft within the medullary canal or even femoral cracking. Furthermore, the range of motion of the femur portion in relation to the acetabular component was insufficient.

In order to overcome the disadvantages of the prior art, numerous experiments were conducted for improving the structure of hip joint prostheses. It was surprisingly found that, with the hip joint prostheses of the present invention, an improved load transfer, an enhanced proximal fixation of the prostheses in the medullary canal, an enhanced range of motion and an improved torsional and axial stability could be provided. Furthermore, a femoral cracking could be prevented due to reduced interference and distinct seating.

### Summary of the Invention

The invention relates to a prosthesis for implantation into a hollow canal of a bone, said prosthesis comprising
- a shaft member having a longitudinal axis and having a distal end, a proximal end, an anterior side, a posterior side, a medial side and a lateral side, said shaft member widening conically from said distal end to said proximal end and from said medial side to said lateral side, and having a proximal locking zone comprising a number of proximal locking surfaces; and
- a neck for receiving a head portion of the prosthesis, said neck extending from said proximal end of said shaft member in an angle (α), relative to said longitudinal axis, and having a peg portion, a first circumferential fillet, a second circumferential fillet and a chamfer;
- wherein the proximal wedge of the proximal locking surfaces is within a range of from 10 to 20 °, relative to the longitudinal axis.

### Brief Description of the Drawings

Reference is now made to the accompanying Figures showing preferred embodiments of the invention. It is noted that the present invention is not restricted to the embodiments shown in the Figures. In the Figures,
Figure 1 shows an anterior elevational view of the hip joint prosthesis of the present invention;
Figure 2 shows a cross-sectional view of the prosthesis of Figure 1 along the line 2 - 2;
Figure 3 shows a cross-sectional view of the prosthesis of Figure 1 along the line 3 - 3;
Figure 4 shows a magnified anterior elevational view of the proximal locking zone of the prosthesis of Figure 1;
Figure 5 shows a magnified lateral elevational view of the proximal locking zone of the prosthesis of Figure 1;
Figure 6 shows a lateral elevational view of the hip joint prosthesis of the present invention; and
Figure 7 shows schematically the proximal wedge of the proximal locking surfaces of the proximal locking zone, wherein Figure 7A shows the proximal wedge of the prosthesis of the present invention while Figure 7B shows the proximal wedge of prostheses of the prior art.

### Detailed Description of the Invention

In the description which follows, it should be understood that any reference to the Figures including references to specific orientations or directions shown in the Figures is not in any way intended to be a limitation of the scope of the present invention.

In the subsequent description, the present invention is explained by taking a hip joint prosthesis as an example, as also the Figures show different examples of a hip joint prosthesis or parts thereof. This should, however, not be understood as restricting the present invention to hip joint prostheses only. The prostheses of the present invention may be configured into many other types of implantable prosthetic devices. For example, the prosthesis of the present invention may be configured as a humeral component of a shoulder prosthesis. Hip joint prostheses are, however, a preferred embodiment of the invention, and they serve as the illustrative example by which the principles and preferred features of the present invention are explained in more detail.

As used in the present specification and claims, the term "proximal" refers to the portion of the prosthesis positioned closest to the heart, while the term "distal" refers to the portion of the prosthesis positioned farthest from the heart. As used in the present specification and claims, the term "anterior" refers to the portion of the prosthesis facing towards the front of the body, while the term "posterior" refers to the portion of the prosthesis facing towards the rear of the body. As used in the present specification and claims, the term "medial" refers to the portion of the prosthesis facing to the center line of the body receiving or holding the prosthesis, while the term "lateral" refers to the portion of the prosthesis facing to one side (left hand side or right hand side) of the body receiving or holding the prosthesis.

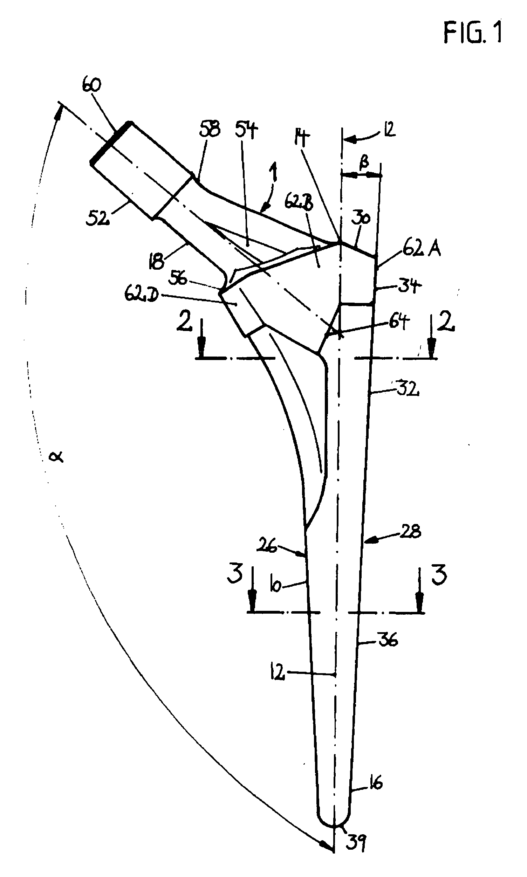

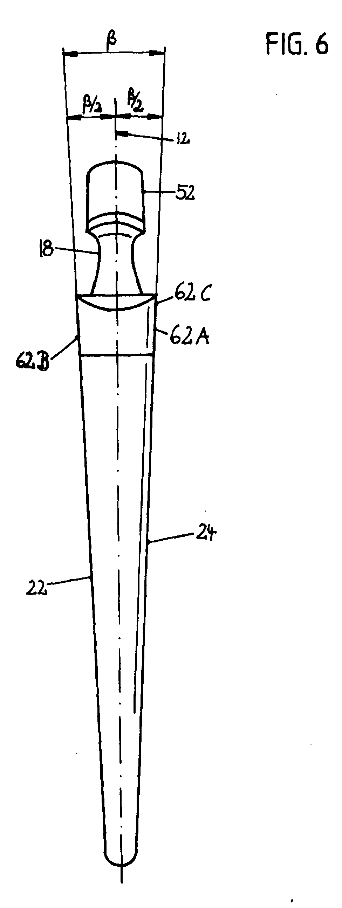

Referring now to Figure 1, there is shown a side elevational view of a hip joint prosthesis according to the present invention designed by the reference numeral 1. The prosthesis 1 of Figure 1 is designed as a hip joint prosthesis. The prosthesis 1 comprises a shaft member 10 having a distal end 16, a proximal end 14, an anterior side 22 (Figure 6), a posterior side 24 (Figure 6), a medial side 26 and a lateral side 28. The prosthesis 1 also comprises a neck 18 designed to receive a head prosthesis. The shaft member 10 has an axis 12 extending longitudinally from the tip of the distal end 16 towards the proximal end 14 of the shaft member 10. The neck 18 extends medially from the proximal end 14 of the prosthesis, and its axis forms an angle (α) with the longitudinal axis 12 of the shaft member 10. In preferred embodiments of the invention, said angle (α) is in a range of from 125 ° to 150 °, more preferably in a range of from 130 ° to 145 °, even more preferably in a range of from 130 ° to 135 °, for example 131 °, 131.5 ° or 132 °.

A spherically shaped driver recess 30 is formed in the proximal end 14 of the stem 10. The driver recess 30 provides an area for the placement of an impaction tool which may be used to drive the prosthesis into the bore formed in the canal of the femur.

Starting at the proximal end 14 and extending distally therefrom is a tapered portion 32 which defines a proximal locking zone 34. The tapered portion 32 extends distally from the proximal locking zone 34 and merges into a conical portion 36. The free end of the conical portion 36 forms the distal end 16 of the shaft member 10, which tapers down from the conical portion 36 to a generally spherical tip portion 39.

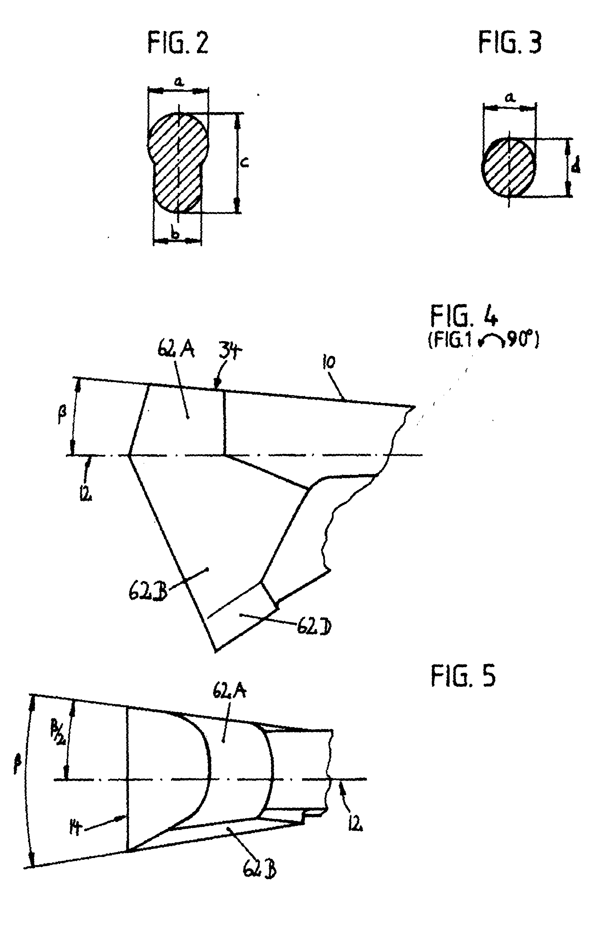

Figures 2 and 3 illustrate the changing cross section of the tapered portion 32 at respective lines 2 - 2 and 3 - 3 in Figure 1. The cross-sectional shape of the tapered portion 32 of the shaft member 10 at line 2 - 2 (Figure 2) presents a greater medial-lateral dimension 38 as compared with the overall anterior-posterior dimension 40 (as measured on the lateral side 28). The cross-sectional shape of the tapered portion 32 changes significantly such that the medial-lateral dimension 38 and anterior-posterior dimension 40 defines an almost rectangularly rounded cross-section at line 3 -3 (Figure 3) approaching the distal end 16 of the tapered portion 32. Also, the anterior-posterior width 42 adjacent the lateral side of the tapered portion 32 becomes substantially greater in width, moving proximally, than the corresponding anterior-posterior width adjacent the medial side.

Referring again to Figure 1, the neck 18 of the prosthesis 1 is tapered and includes a peg portion 52. The proximal end 14 of the shaft member 10 merges into a distal end 54 of the neck 18 forming a first circumferential fillet at 56. The neck 18 converges proximally as it merges into the tapered portion 52 forming a second circumferential fillet at 58. The tapered portion 52 is slightly tapered in the distal to proximal direction and includes a chamfer 60 at the proximal end of the tapered portion 52. The tapered portion 52 is adapted to receive the femoral bearing head portion of the prosthesis (not shown).

Still referring to Figure 1, there is illustrated the proximal locking zone 34 portion of the shaft member 10. The proximal locking zone 34 comprises a number of, for example, four proximal locking surfaces 62A, 62B, 62C, 62D, ...., which extend circumferentially around the proximal portion of the tapered portion of the shaft member 10. Proximal locking surfaces 62A, 62B, 62C, 62D, ...., step down at circumferential step 64 to meet the remaining portion of tapered portion 32.

The details of the proximal locking zone 34 are best illustrated by referring to Figure 4. Locking surface 62A extends between locking surfaces 62B and 62C (not visible) and presents a conically shaped surface on the lateral side of the prosthesis 1. Locking surface 62 D extends between locking surfaces 62B and 62C and presents a curved surface on the medial side of the prosthesis 1. Locking surfaces 62B and 62C are planar and are located, respectively, on the anterior and posterior sides of the prosthesis.

The anteriorly and posteriorly located locking surfaces 62B and 62C each lie in a plane that defines a locking angle or taper β which, in preferred embodiments of the invention, ranges between 10 and 20 °, more preferably between 12 and 18 °, for example 12 °, 13 °, 14 ° or 15 °, relative to the longitudinal axis 12 (see also Figure 5). The angles β/2 are best illustrated in Figure 5 where the prosthesis 1 has been rotated by 45 ° around the longitudinal axis 12.

Referring again to Figure 4, the conically shaped surface presented by the laterally located locking surface 62A tapers, in preferred embodiments of the invention, at the same locking angle β as locking surfaces 62B and 62C. In other words, in preferred embodiments of the invention, the locking angle β preferably ranges between 10 ° and 20 °, more preferably between 12 ° and 18 °, most preferably between 13 ° and 15 °, for example 14°. The curved surface presented by the medially located locking surface 62D follows the general taper of the medial portion 26 of the shaft member segment 10 and presents a slightly proud surface. This configuration provides an uninterrupted continuum of locking surfaces 62B and 62C.

Referring now to Figure 6, there is shown the lateral side of the prosthesis 1 of the present invention. Figure 6 clearly illustrates the angle β defined by the proximal locking surfaces 62B and 62C and the anterior-posterior tapering of the remaining portion of the proximal locking zone.

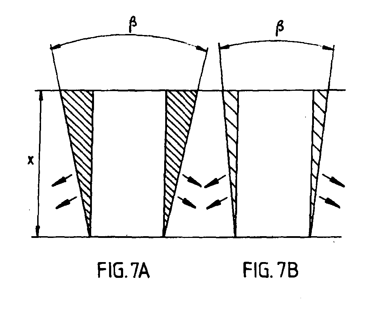

A prosthesis of the present invention showing the above values of the taper of the locking surfaces 62B, 62D much better focusses the load transfer in the proximal femur than in prostheses of the prior art having smaller taper values. The stump taper of the prosthesis proximal part displaces much more bone material when sinking into the bone canal by a certain distance (e. g. the distance of x, see Figure 7A) than a prosthesis having a steeper taper, as usual in the prior art. The prosthesis including the stump taper locks proximally sooner, resulting in an earlier introduction of loading forces in the proximal femur. In addition, the risk of fissures of the bone material, e. g. in the femur, are substantially reduced due to the fact that reduced hoop stresses occur. Furthermore, it is of utmost advantage that, due to a stump proximal taper, there is an increased resistance against subsidence of the prosthesis after the hip replacement surgery. In addition, the range of motion of the femur portion of the novel hip prosthesis in relation to the acetabular component is substantially enhanced.

In the present invention, particularly in the exemplary embodiment of the novel hip prosthesis, it is of further advantage that the shaft member 10 anchored in the medullary canal is reliably closed or sealed, respectively, against the immigration of wear particles which might otherwise migrate from the head (articulating in the socket formed in the acetabular component) into the portion in the medullary canal anchoring the shaft member 10.

The prosthesis of Figures 1 to 6 is intended to be implanted by a press-fit engagement which does not utilize a cement for locking into place. The term "press-fit" as used in the present specification and claims is intended to mean a mechanical engagement formed by two components (e. g. the bone and the prosthesis material), at least one of which is deformable, where the adjacent boundary lines of the two components overlap and interface with each other such that the two components must be forced into a position adjacent each other which produces a locking force developed over the area of contact between the two components.

In preferred embodiments of the present invention, one or more than one proximal locking surface(s) 62A, 62B, 62C, 62D ..., is/are grit blasted in order to provide a surface which is reliably engaged with the inner wall of the medullary canal. In yet another preferred embodiment of the invention, a layer or more than one layer of a bio-active material or "synthetic bone" material is/are deposited on the proximal locking surfaces 62A, 62B, 62C, 62D, ..., which allow the prosthesis 1 to become permanently attached via a mechanism which includes growth of the natural bone tissue within the cortex of the bone into the layer(s) of bio-active material. In a preferred process, the synthetic bone material is plasma-sprayed on the prosthesis 1. The preferred choice of synthetic bone material can be any well known ceramic comprising at least one artificial apatite in the form of hydroxyapatite (CaH(PO₄)₂). This material can be applied in a single layer or in multiple layers, and in case of multiple layers, the layers can be composed of different materials. The layer(s) of synthetic bone material is/are designed to be highly compatible with natural bone tissue.

The prosthesis of the present invention can be manufactured from any known metallic material. In preferred embodiments of the invention, the prosthesis may be made of well known metals or metal alloys. Particularly preferred examples are titanium alloys, cobalt-chromium alloys as well as alloys containing aluminum. Materials which are of utmost preference are titanium-aluminum alloys, for example the alloy Ti-A16-V4. The prosthesis may be manufactured by forging, casting and/or machining operations or any other well-known technique.

An implantation of the prosthesis of the present invention may be effected by any process known to a person of usual skill in this field. For suitable implantation processes, reference may be made to the documents U.S. Patent No. 4,728,334 and U.S. Patent No. 5,863,295, which are incorporated herein by reference.

In a preferred embodiment, the invention also relates to a process for inserting a prosthesis into a bone and at least in part engaging the prosthesis with the material of the bone. The process includes the steps of generating a canal in the bone, said canal being, suitable in shape and size to receive at least a part of the prosthesis, inserting at least said part of the prosthesis into the canal thus generated, drive at least said part of the prosthesis into said canal by means of a suitable impaction tool so as to engage at least a part of one or more than one of outer walls of said part of the prosthesis inserted into the canal with at least a part of the inner walls of the canal. By such a process, there may be achieved, according to the present invention, a rigid and stable mechanical engagement of the prosthesis to the bone, if the prosthesis is a prosthesis as described above in detail.

In even more preferred embodiments of the invention which, however, are not restricting the scope of the invention, the process is a process of employing a hip prosthesis, and the bone to which the prosthesis of the present invention preferably is engaged is a femur, for example a femur of a human. In another preferred embodiment of the process of this invention, the step of inserting the prosthesis into the bone and engaging at least a part thereof with the bone material is a step of pacing the impaction tool to a part of the prosthesis allowing the application of a mechanical force in a direction substantially corresponding to the axis of that part of the prosthesis which is to be engaged to the canal of the bone. Even more preferred, an impaction tool is placed into a driver recess formed at a location of the prosthesis which is distal from the end of the prosthesis to be inserted into the bone and concentrically with the axis of the prosthesis. This preferred embodiment allows the part of the prosthesis of the present invention to be inserted into the bone and at least a sufficiently large surface part thereof to be brought in close engagement, more preferably into a "press-fit" engagement, with the bone, for example with the inner canal formed in a bone, for example in the femur of a human. By this process, a rigid fixation of said part of the prosthesis within the bone can be achieved. In an utmost advantageous way, a locking force is produced between the bone material and the prosthesis material so that a subsidence of the prosthesis into the bone after the surgery is no longer observed.

Finally, the invention also relates to the use of a prosthesis as in detail described above, in particular to the use of a prosthesis having the above preferred features, for replacing a part of a natural joint. In a preferred embodiment, the invention relates to the use as indicated above, wherein the natural joint is a hip joint, preferably the hip joint of a human, although other joints (e. g. shoulder) or joints of animals (for example dogs or cats) may be replaced by the prosthesis of the present invention. In a most preferred embodiment of the invention, the prosthesis of the present invention may be used for replacing the femural part of a hip joint, more preferably the femural part of a hip joint of a human.

The above preferred embodiments of the invention are given for illustrative purposes only, and it is not intended that they are used to restrict the scope of the present invention which is given in the appended claims.

## Claims

1. A prosthesis (1) for implantation into a hollow canal of a bone, said prosthesis comprising
- a shaft member (10) having a longitudinal axis (12) and having a distal end (16), a proximal end (14), an anterior side (22), a posterior side (24), a medial side (26) and a lateral side (28), said shaft member (10) widening conically from said distal end (16) to said proximal end (14) and from said medial side (26) to said lateral side (28), and having a proximal locking zone (34) comprising a number of proximal locking surfaces (62A, 62B, 62C, 62D, ....); and
- a neck (18) for receiving a head portion of the prosthesis, said neck (18) extending from said proximal end (14) of said shaft member (10) in an angle (α), relative to said longitudinal axis (12), and having a peg portion (52), a first circumferential fillet (56), a second circumferential fillet (58) and a chamfer (60);
- wherein the proximal wedge of the proximal locking surfaces (62B, 62D) is within a range of from 10 ° to 20 °, relative to the longitudinal axis (12).

2. The prosthesis (1) of claim 1, which is a hip prosthesis for implantation into the medullary canal of a femur, wherein the neck (18) receives a femoral head prosthesis.

3. The prosthesis (1) of claim 1 or claim 2, wherein the angle (oc) between the longitudinal axis (12) of the shaft member (10) and the neck (18) is in a range of from 125 ° to 150 °.

4. The prosthesis (1) of any of claims 1 to 3, wherein the proximal taper of the proximal locking surfaces (62B, 62D) is within a range of from 10 to 20 °, preferably within a range of 12 ° to 18 °, more preferably within a range of 13 ° to 15 °.

5. The prosthesis (1) of any of claims 1 to 4, wherein the axis of the neck (18) forms an angle in the range of 125 ° to 150 °, more preferably in the range of 130 ° to 145 °, most preferably in the range of 130 ° to 135 °, with the longitudinal axis (12) of the shaft member (10) of the prosthesis (1).

6. The prosthesis (1) of any of claims 1 to 5, wherein the femur portion has an enhanced range of motion in relation to the acetabular component.

7. The prosthesis (1) of any of claims 1 to 6, wherein one or more than one of said proximal locking surfaces (62A, 62B, 62C, 62D ...) is/are grit blasted.

8. The prosthesis (1) of any of claims 1 to 6, wherein one or more than one of said proximal locking surfaces (62A, 62B, 62C, 62D ...) comprise(s) one or more than one layer of one or more than one material which promotes growth of natural bone tissue into said layer and/or which enhances the attachment of the prosthesis (1) to the bone material.

9. The prosthesis (1) of claim 8, wherein two or more layers may comprise more than one material.

10. The prosthesis (1) of claims 8 or 9, wherein said layer(s) of material comprise a synthetic bone material coating said proximal locking surface (62A, 62B, 62C, ...), preferably wherein said layer(s) of synthetic bone material comprise(s) hydroxyapatite.

11. The prosthesis (1) of any of the claims 1 to 10, wherein the material of the prosthesis (1) is a metal or metal alloy, preferably an alloy comprising Ti, more preferably an alloy comprising Ti, Al and V.

12. A process for inserting a prosthesis into a bone and at least in part engaging the prosthesis with the material of the bone by generating a canal in the bone, said canal being suitable in shape and size to receive at least a part of the prosthesis, inserting at least said part of the prosthesis into the canal thus generated, drive at least said part of the prosthesis into said canal by means of a suitable impaction tool so as to engage at least a part of one or more than one of outer walls of said part of the prosthesis inserted into the canal with at least a part of the inner walls of the canal, thereby achieving a rigid and stable mechanical engagement of the prosthesis to the bone, wherein the prosthesis is a prosthesis of any of claims 1 to 11.

13. The process of claim 12, wherein the prosthesis is a hip prosthesis, and the bone is a femur.

14. The process of claim 12 or claim 13, wherein the impaction tool is placed to a part of the prosthesis allowing the application of a mechanical force in a direction substantially corresponding to the axis of that part of the prosthesis which is to be engaged to the canal of the bone.

15. The process of any of claims 12 to 14, wherein the impaction tool is placed into a driver recess formed at a location of the prosthesis which is distal from the end of the prosthesis to be inserted into the bone and concentrically with the axis of the prosthesis.

16. Use of a prosthesis as claimed in any of the claims 1 to 11 for replacing a part of a natural joint.

17. The use according to claim 16, where the natural joint is a hip joint, preferably a hip joint of a human.

18. The use according to claims 16 or 17, wherein the part replaced by the prosthesis is the femural part of a hip joint, preferably of a hip joint of a human.
